# EUROPEAN PATENT SPECIFICATION

(11) **EP 4 515 529 B1**
(45) Date of publication and mention of the grant of the patent: **12.08.2026**
(21) Application number: 23721758.3
(22) Date of filing: 27.04.2023
(51) Int. Cl.: G10L 15/01, A61B 5/00, G10L 15/26

(54) **ESTIMATING THE ACCURACY OF AUTOMATICALLY TRANSCRIBED SPEECH WITH PRONUNCIATION IMPAIRMENTS**
SCHÄTZEN DER GENAUIGKEIT VON AUTOMATISCH TRANSKRIBIERTER SPRACHE MIT AUSSPRACHESCHWÄCHEN
ESTIMATION DE LA PRÉCISION DE LA PAROLE TRANSCRITE AUTOMATIQUEMENT AVEC DES TROUBLES DE LA PRONONCIATION

(30) Priority: 29.04.2022 EP 22170713
(43) Date of publication of application: 05.03.2025
(73) Proprietor: F. Hoffmann-La Roche AG, 4070 Basel (CH)
(72) Inventor: KOSEK, Michal Kajetan, 31-223 Kraków (PL); ULLMANN, Raphael Marc, 8050 Zürich (CH)
(74) Representative: Betten & Resch
(86) International application number: PCT/EP2023/061163
(87) International publication number: WO 2023/209115

(56) References cited:
- WO-A1-2019/207573
- US-B1- 10 854 190
- COX S J ET AL: "TOWARDS SPEECH RECOGNIZER ASSESSMENT USING A HUMAN REFERENCE STANDARD", COMPUTER SPEECH AND LANGUAGE, ELSEVIER, LONDON, GB, vol. 12, no. 4, 1 October 1998 (1998-10-01), pages 375 - 391, XP000883469, ISSN: 0885-2308, DOI: 10.1006/CSLA.1998.0109
- HANWU SUN ET AL: "Investigations into the relationship between measurable speech quality and speech recognition rate for telephony speech", ACOUSTICS, SPEECH, AND SIGNAL PROCESSING, 2004. PROCEEDINGS. (ICASSP ' 04). IEEE INTERNATIONAL CONFERENCE ON MONTREAL, QUEBEC, CANADA 17-21 MAY 2004, PISCATAWAY, NJ, USA,IEEE, PISCATAWAY, NJ, USA, vol. 1, 17 May 2004 (2004-05-17), pages 865 - 868, XP010717766, ISBN: 978-0-7803-8484-2

## Description

### TECHNICAL FIELD

The present application generally relates to the field of measuring an accuracy of pronunciation of human speech.

### BACKGROUND

Certain disorders, such as autism spectrum disorder (ASD) can result in unclearly articulated human speech.

US 10,854,190 B1 refers to evaluating transcription accuracy. A system normalizes a first transcription of an audio file and a baseline transcription of the audio file. The baseline transcription is used as an accurate transcription of the audio file. The system further determines an error rate of the first transcription by aligning each portion of the first transcription with the portion of the baseline transcription, and assigning a label to each portion based on a comparison of the portion of the first transcription with the portion of the baseline transcription .
The publication S.J.Cox et al. "TOWARDS SPEECH RECOGNIZER ASSESSMENT USING A HUMAN REFERENCE STANDARD" in COMPUTER SPEECH AND LANGUAGE, ELSEVIER, LONDON, GB, pages 375-391, 01-10-1998 discloses the use of a human reference standard to assess the performance of speech recognizers, so that the performance of a recognizer could be quoted as being equivalent to the performance of a human hearing speech which is subject to X dB of degradation.
The publication H.Sun et al. "Investigations into the relationship between measurable speech quality and speech recognition rate for telephony speech" in Proceedings of ICASSP 2004, 17-21 may 2004 discloses an investigation of a possible relationship between the performance of a telephony speech recognition system and the method for objective speech quality assessment of ITU-T recommendation P.862.
The international patent application WO2019207573A1 discloses techniques for assessing a speech/lingual quality of a subject.

An assessment of intelligibility of human speech is usable in the context of automated speech recognition (ASR) and transcription. For example, it can be desirable to detect human speech that is unclearly articulated and exclude it from speech transcripts and the further processing thereof. In a further example, a measure for intelligibility of human speech can be useful when evaluating an ASR service, for example to determine whether malperformance of ASR through the service is due to pathological speech input or due to limitations of the ASR service itself. The terms ASR service, ASR system, and ASR technology are used interchangeably herein unless explicitly mentioned.

Moreover, an assessment of intelligibility of human speech is usable in the context of diagnosis, clinical study or therapy. For example, it can be desirable to detect human speech that is unclearly articulated as a potential marker of a motor, behavioral or cognitive disorder, and to track its change over time to evaluate disorder progression, treatment response, or to help affected individuals monitor and adapt their speech.

In the field of ASR, recognition performance is commonly measured in terms of a word error rate (WER). The WER is a standard metric which measures a mismatch between the output of an ASR service in response to a speech recording and a corresponding human transcript of the same speech recording, i.e., a reference transcript, which is a transcript created by a human listener. The WER is computed as the edit distance between the ASR output and the human transcript (i.e., the number of words that need to be inserted, deleted or substituted in order to make the ASR output identical to the manual human transcription), divided by the total number of words in the human transcript. The WER is a rational number value greater than or equal to 0. A WER of 0 specifies a perfect match of the ASR output with the human transcript, whereas a WER of 1 indicates that the number of edits (insertions, deletions or substitutions) that are needed to make the ASR output equal to the human transcript is as large as the number of words in the human transcript. A WER value greater than 1 is possible if, for example, the ASR output contains many more words than the human transcript, i.e., the number of deletions needed is greater than the number of words in the human transcript.

The WER is an objective measure of the accuracy of a speech transcription. Objective in this context means that the measure is independent of the implementation of a specific ASR service and usable to compare recognition accuracy across different ASR services. The WER is a function of the input and the output of an ASR service. In other words, different ASR services that create the same output from the same input have identical WERs.

The determination of the WER requires a reference transcript. At least for speech for which such a reference transcription is not available or known upfront, the preparation of a reference transcript by a human is a laborious process and is therefore inconvenient in practice.

Unclear pronunciation in a speech input can reduce the ability of an ASR service to produce a correct transcript, i.e., to achieve a low WER. Conversely, a high WER can be indicative of unclear or uncommon pronunciation of the speech input, poor audio quality (noise, reverberation), unexpected pronunciation (accents), unexpected words (vocabulary, slang), or unexpected sentences (e.g. the utterance "the sky is boo" would be transcribed as "the sky is **blue"** by most ASR systems).

Many ASR systems produce a so-called confidence score for each word in an automatically generated transcript. The confidence score is typically a rational number between 0 and 1. The confidence score specifies a probability with which a given ASR service determined the respective word as a transcription of the speech input. A confidence score of 1 indicates that the ASR is certain about the result of the recognition, whereas a confidence score close to 0 indicates that result of the recognition is very uncertain, such as randomly chosen. Analogous to the confidence score of individual words, an ASR system may determine an overall confidence score for a sequence of words of an entire sentence or transcript. For example, confidence scores for words may be primarily driven by the acoustics of the speech input; the algorithmic piece for this is the Acoustic Model. In contrast, confidence scores for sentences would typically consider the likelihood of the *word sequence* in a language, as determined by a Language Model. As an example, a language model will make the decision between "recognize speech" and "wreck a nice beach" (similar acoustics but different words) based on the surrounding context. More recently, many ASR systems have moved to so-called End-to-End architectures, where there is no explicit separation between Acoustic and Language Models anymore. In summary, the overall confidence score for a transcript can be different from the mean of the confidence scores of individual words.

The overall confidence value is a measure for the accuracy of an automatic transcription since the value is determined by the ASR service itself based on the speech input, the internal implementation of the ASR, its internal ASR parameters, and related statistical models. Therefore, the confidence score is a function of the speech input and internals of an ASR system. In other words, different ASR systems that create the same output from the same input can still have different overall confidence scores for their respective recognition process.

Some conventional techniques for estimating the reliability of automatic transcripts are based on the confidence score. To mitigate the bias for specific ASR implementations and their internal parameters, other conventional techniques combine features from multiple different ASR systems to produce alternative confidence scores and/or transcriptions of one and the same speech input recording to estimate an improved measure of ASR transcription accuracy.

Since the determination of a WER for a recognition or transcription task is afflicted with the above problem of manually determining a reference transcription, the overall confidence score is frequently used as an estimation or approximation of the true WER. However, such estimation may be inaccurate as will be shown herein. In some cases, depending on the ASR service and its implementation, there is little correlation between the true WER and the overall confidence score.

It is therefore an objective of the technology described herein to provide an accurate estimator for the WER in automated speech recognition that is robust, i.e., not dependent on a specific ASR implementation, and that does not require manual creation of a reference transcription.

Furthermore, as explained above, the confidence score is an approximation of a WER and is dependent on the specific implementation of an ASR service.

It is a further objective of the present technology to provide an assessment of speech intelligibility for a given speech input that is largely independent of the specifics of different ASR technologies and their implementation.

### SUMMARY

A simplified summary of some embodiments of the disclosure are provided in the following to give a basic understanding of these embodiments and their advantages. Further embodiments and technical details are given in the detailed description presented below.
The invention provides a computer-implemented method for determining an accuracy of automatically transcribed pathological speech according to claim 1, a data processing device according to claim 12, a computer program according to claim 13 and a computer-readable medium according to claim 14. The dependent claims specify embodiments thereof.

The computer-implemented method according to claim 1 includes comparing the first transcript with the second transcript to quantify a mismatch between the first transcript and the second transcript, wherein quantifying a mismatch between the first transcript and the second transcript includes determining a difference as a measure of accuracy of the first transcript. An advantage of this method is that the mismatch has a high correlation to the presence of pronunciation errors in the original speech recording. This correlation reflects the presence of pronunciation errors more accurately than an overall confidence score provided by conventional ASR systems. Unlike such overall confidence score, the mismatch is largely independent of the underlying ASR system that is used.

In some embodiments, the perturbation is one or more of: additive noise, multiplicative noise, reverberation, decomposing the original speech recording into a parametric representation and re-synthesizing it after varying one or more of the parameters, and speech-like noise obtained by combining fragments of speech from one or more different voices. This aspect has the advantage that the determined mismatch reliably reflects words that are unclearly pronounced, since the specific types of perturbation tend to specifically impact the recognition of conventional ASR systems for words that are unclearly pronounced. In other words, the choice of perturbation is suitable to increase the level of uncertainty of recognition in current ASR systems exactly for those words in the original audio recording that are unclearly pronounced.

In some embodiments, the signal-to-noise ratio when combining the perturbation with the original speech recording is larger than 0 and lower than 40 dB. This aspect has the advantage that the effect of perturbation is limited in a meaningful degree, so that words that are clearly pronounced can still be reliably recognized by the ASR, and for words with unclear pronunciation, recognition is hampered, such that ASR will more likely err on these unclearly pronounced words. Too much perturbation would deteriorate the recognition rate of clearly pronounced words, too little perturbation would have too little impact on deteriorating the ASR's recognition accuracy of unclearly pronounced words in the original audio recording.

In some embodiments, the signal-to-noise ratio is determined by: performing ASR on each of one or more test speech recordings for which a word error rate is known to obtain one or more original test transcripts; iteratively performing the steps of combining a perturbation with each of the test speech recording at a test signal-to-noise ratio to obtain one or more perturbed test speech recordings; performing ASR on each of the perturbed test speech recordings to obtain one or more perturbed test transcripts, comparing the original test transcripts with the respective perturbed test transcripts to determine a transcript mismatch measure; varying the test signal-to-noise ratio based on the transcript mismatch measure; until a maximum correlation between the transcript mismatch measure and the known word error rate is achieved, wherein the signal-to-noise ratio is determined based on the current test signal-to-noise ratio. This has the advantage that an ideal signal-to-noise ratio can be determined according to which the perturbation is combined into the original audio speech signal.

Quantifying a mismatch between the first transcript and the second transcript includes determining a difference as a measure of accuracy of the first transcript.

In some embodiments, the automatic speech recognition on the original and on the perturbed speech recording is performed using a same ASR technology. In that manner, a mismatch due to different recognition algorithms in different ASRs can be avoided. Such mismatch is undesirable and would be misleading in the above described determination of pronunciation errors.

In some embodiments, the method further comprises: comparing the measure of transcript accuracy with a measure of transcript accuracy obtained from prior speech recordings of the same person to assess a change in unclear pronunciation of the person and/or to track a level of language development of the person. In that manner, a person can track the development and quality of her own pronunciation over time.

In this embodiment, optionally, a change in a cognitive status of the person is assessed based on a change in unclear pronunciation. Also, optionally, one or more of a cognitive or behavior disorder of the person and autism spectrum disorder, ASD, are tracked.

In some embodiments, the method further comprises comparing the measure of transcript accuracy with a measure of transcript accuracy obtained from speech recordings from one or more persons different from the person to determine unclear pronunciation as a pronunciation impairment of the person and/or to track one or more of a level of language development or pronunciation accuracy of the person and a motor or behavioral impairment affecting pronunciation of the person, wherein the one or more persons different from person preferably belong to age-, gender-, and education-matched healthy controls. In that manner, a person compares the quality of her own pronunciation with a control group and tracks the development of her own pronunciation over time.

In some embodiments, the unclear pronunciation relates to word-finding difficulties, other types of language attrition, or incoherent speech.

In some embodiments, the speech is pathological due to pronunciation impairments of the person.

In some embodiments, the pronunciation impairments relate to individual words and/or comprise one or more of hypoarticulation, hyperarticulation, slurred speech, stutter, and mumble.

The technology disclosed herein can also be embodied in a data processing device comprising a processor adapted to perform the steps of the method for determining an accuracy of automatically transcribed pathological speech according to the methods specified hereinabove.

The technology disclosed herein can also be embodied in a computer program comprising instructions that, when the program is executed by a computer, cause the computer to carry out the steps of the method for determining an accuracy of automatically transcribed pathological speech according to the methods specified hereinabove.

The technology disclosed herein can also be embodied in a computer-readable medium comprising instructions that, when executed by a computer, cause the computer to carry out the method for determining an accuracy of automatically transcribed pathological speech according to the methods specified hereinabove.

The present technology provides one or more the advantages and technical effect described hereinabove.

The foregoing general description and the following detailed description are exemplary and explanatory only and are not restrictive of the invention as claimed.

### BRIEF DESCRIPTION OF THE DRAWINGS

The foregoing summary as well as the following detailed description of preferred embodiments are better understood when read in conjunction with the appended drawings. For illustrating the invention, the drawings show exemplary details of systems, methods, and experimental data. The information shown in the drawings are exemplary and explanatory only and are not restrictive of the invention as claimed. In the drawings:
Fig. 1 is a flowchart showing a computer-implemented method for estimating an accuracy of automatically transcribed pathological speech.
Fig. 2 shows a model of determining ASR prediction accuracy.
Fig. 3A-C are plots of statistical data showing word error rates, WER, per recording vs a conventional mean confidence score per recording created by three different ASR services.
Fig. 4A-C are plots of statistical data showing WER per recording vs WER between two automatic transcripts generated according to the present disclosure using three different ASR services.
Fig. 5 is a chart plot comparing the prediction of transcription accuracy based on conventional methods of mean confidence scores with the method according to the present disclosure for three different ASR services.
Fig. 6 is a schematic block diagram of a data processing device adapted to perform methods for determining an accuracy of automatically transcribed pathological speech according to at least one example embodiment.

### DETAILED DESCRIPTION

The present disclosure relates to methods and systems for determining and measuring the intelligibility of speech, in particular pathological speech, to the ears of a human listener as well as to the "ears" of a speech-to-text system, also referred to as automatic speech recognition, ASR, system herein. The described technology can produce a measure of unclear pronunciation, which can be clinically relevant in itself, i.e., even if there is no intention to do anything else with the transcripts created by an ASR service.

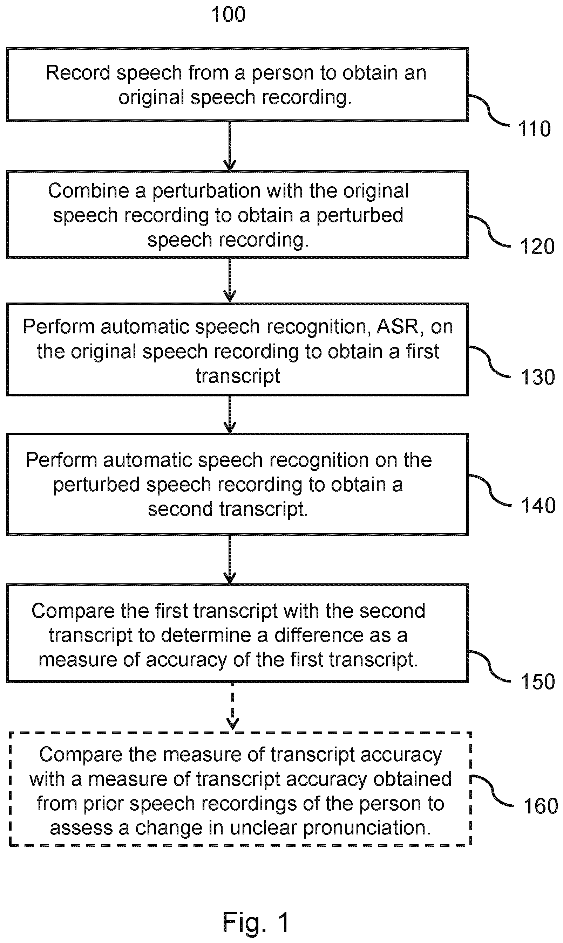

Fig. 1 shows a flowchart of a method 100 for estimating an accuracy of automatically transcribed pathological speech. The method 100 is not limited to this purpose and can also be used to provide an objective assessment of speech intelligibility. Speech is pathological due to pronunciation impairments of the person 210 who is speaking, wherein pronunciation impairments relate to individual words and/or comprise one or more of hypoarticulation, hyperarticulation, slurred speech, stutter, and mumble.

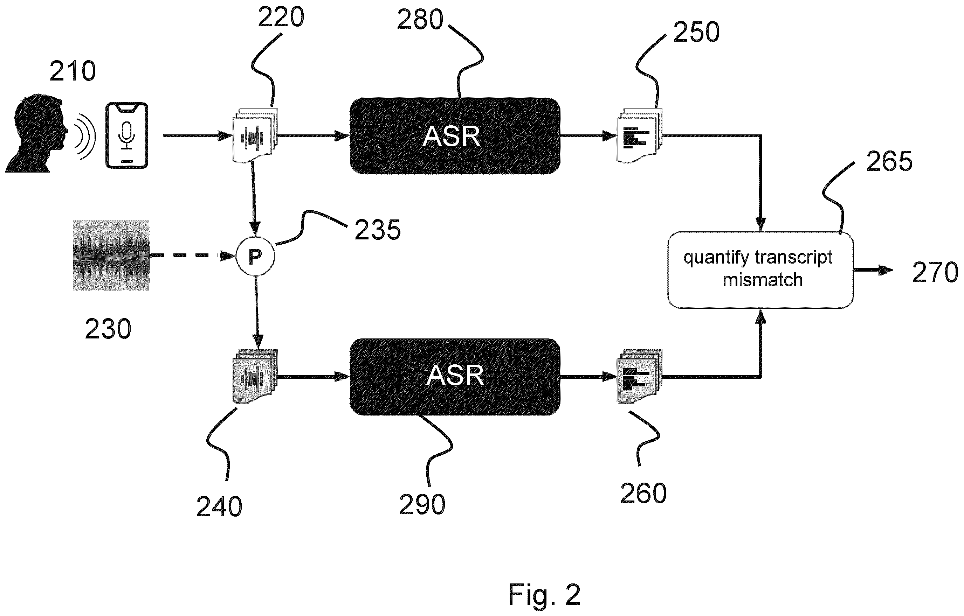

Fig. 2 shows a model of determining ASR prediction accuracy. The elements of Fig. 2 will be described in the context of the method 100 described in the following.

The method 100 comprises the step of recording 110 speech from a person 210 to obtain an original speech recording 220. Conventional audio technology may be suitable to implement this task.

The method 100 further comprises the step of combining 120 a perturbation 230 with the original speech recording 220 to obtain a perturbed speech recording 240.

The perturbation 230 can be an audio signal independent of the original speech recording 220 and can be pre-recorded or generated on the fly. The perturbation 230 may take the form of additive noise, which is noise that is added to the original speech recording 220. The noise can take any form of additive noise, for example color noise. Fig. 2 illustrates component 235 for audio signal processing that performs the step of combining 120 the signal of the original audio recording 220 with the signal of the perturbation 230. Component 235 is indicated in Fig. 2 with a "P" since it causes perturbation to be added to the original audio recording 220. Note that the perturbation is indicated in the Fig. 2 as a separate signal 230, which is an input to component 235. The arrow between the signal 230 and the component 235 is dashed, which indicates that such a separate signal 230 is optional for the step of combining 120 the original speech recording 220 with a perturbation. In some embodiments, the signal 230 may be a signal independent of the original audio signal 220. In some embodiments, the signal 230 may be derived from the original audio signal 220. In some embodiments, the perturbation through component 235 may solely be based on the original signal 220 taken as input to the perturbation. Examples of these cases will be given in the following. For all those cases, we refer to combining 120 a perturbation 230 with the original speech recording 220 to obtain a perturbed speech recording 240.

While additive noise is a preferred form of noise, the perturbation 230 may also be an audio signal derived from the audio signal of the original speech recording 220, for example multiplicative noise. The noise can also take the form of a reverberation of the original speech recording 220 or noise obtained by decomposing the original speech recording 220 into a parametric representation and re-synthesizing it after varying one or more of the parameters such as vocoding of the original speech recording 220 or some other speech recording. This can be thought of as decomposing a musical performance into scores for each instrument (the parametric representation), changing the notes, tempo, scale, instruments etc., and recording an orchestra performing those modified scores (the re-synthesized recording).

The perturbation 230 can also be speech-like noise obtained by combining fragments of speech from one or more different voices. In one example, such speech-like noise can be babble speech or multi-talker babble speech.

To summarize, the general approach of the combining in step 120 is to distort or perturb the speech recording. Such distortion or perturbation could be achieved through additive noise, which is preferred, but also through multiplicative noise, reverberation, or speech decomposition and re-synthesis methods such as vocoding.

The idea behind and the reason for introducing the perturbation 230 and combining 120 the same with the original speech recording 220 is that the words in the original speech recording 220 that are impacted the most by the described perturbation 230 are the ones that were not clearly pronounced and hence are the most difficult to be recognized by an ASR system.

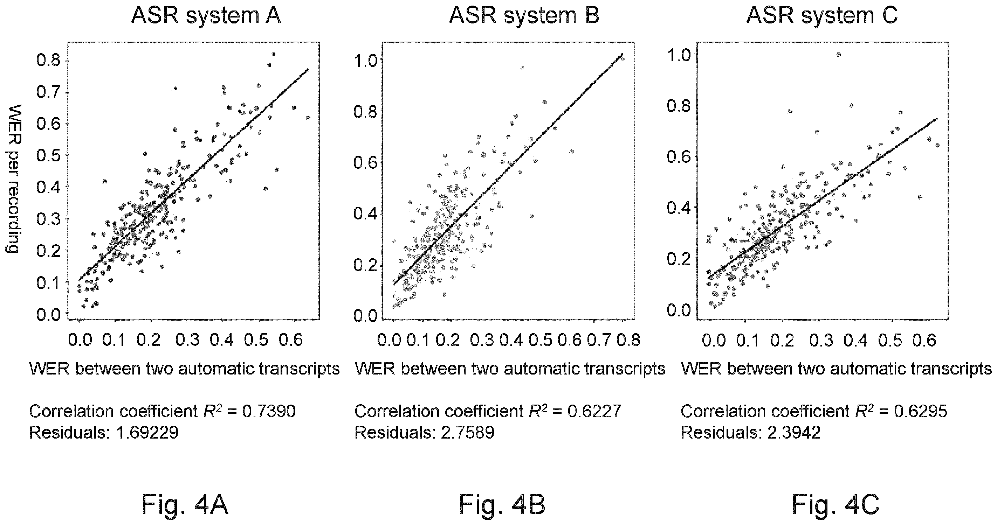

The combining 120 of the perturbation with the original speech recording 220 may be done at certain signal-to-noise ratio (SNR). Preferred SNR values are larger than 0 dB and lower than 40 dB (Decibel). This range is primarily for a perturbation that is additive speech-like noise, for other types of perturbation a specific range could be broader. In the experimental data shown in Fig. 4A, 4B, and 4C, an SNR value of 20 is used. An iterative process of determining a suitable SNR value is described herein at the end of the discussion of the method 100.

The method 100 further comprises the step of performing 130 automatic speech recognition, ASR, on the original speech recording 220 to obtain a first transcript 250. The transcript is a text string including one or more words corresponding to the speech in the original speech recording. The method is independent of the particular ASR technology 280 used for this step, so that any present or future ASR technology may be used to perform step 130.

The method 100 further comprises the step of performing 140 automatic speech recognition on the perturbed speech recording 240 to obtain a second transcript 260. The ASR technology 290 used in this step 140 is preferably identical to the ASR technology 280 used to in step 130.

ASR used in steps 130 and 140 may be implemented in a distributed computing environment, such as by cloud computing service. In such a scenario, the steps 130 and 140 may involve transferring the respective speech recording to a cloud computer, performing ASR in the cloud, and receiving the resulting transcription from the cloud.

The method 100 further comprises the step of comparing 150 the first transcript 250 with the second transcript 260 to quantify a mismatch between the first transcript 250 and the second transcript 260. Said quantifying a mismatch includes determining a difference as a measure of accuracy 270 of the first transcript 250. Component 265 for quantifying a mismatch between the transcripts is shown in Fig. 2 and may be implemented using ordinary string processing. Comparing 150 the first transcript 250 and second transcript 260 includes quantifying a mismatch between the first transcript 250 and the second transcript 260. Specifically, in one example, a difference between the two transcripts can be determined as numerical value based on a pairwise comparison of pairs of words. Therein, each word of a pair is taken from each sequence, wherein the difference is a counter of words in the transcription of the original speech recording, i.e., the first transcription that differed from the corresponding word in the transcription of the perturbed speech recording, i.e., the second transcription. The comparison may detect and handle a situation where a word in one transcript is absent in the other transcript and count such situation, for example, as if a pair of differing words was encountered. A measure of difference can be the total number of differences as well as a relative value such as a fraction of words of the entire transcript for which differences are identified during the comparison 150. Such measure of difference is also referred to as transcript mismatch number or transcript mismatch ratio herein.

A suitable SNR value for the step of combining 120 can be determined, for example, iteratively and up front, by using multiple recordings, e.g., test recordings, for which the WER obtained in combination with a particular ASR service is known. The SNR value is varied in the iterative optimization process such as to maximize the correlation and/or to minimize the error between our "transcript mismatch" measure, and the actual word error rates of the first transcripts. Essentially, the process is using a set of test recordings, e.g., at least 20, and varying the SNR until the obtained measure shows the best agreement with the actual ground truth transcription accuracy or intelligibility.

Thus, an "ideal" SNR is determined, for example, by the steps of performing ASR on each of one or more test speech recordings for which a WER is known to obtain one or more original test transcripts; iteratively performing the steps of combining a perturbation with each of the test speech recording at a test signal-to-noise ratio to obtain one or more perturbed test speech recordings and performing ASR on each of the perturbed test speech recordings to obtain one or more perturbed test transcripts; comparing the original test transcripts with the respective perturbed test transcripts to determine a transcript mismatch measure; varying the test SNR based on the transcript mismatch measure; until a maximum correlation between the transcript mismatch measure and the known WER is achieved, wherein the SNR is determined based on the current test SNR.

The method 100 optionally includes the further step of comparing 160 the measure of transcript accuracy 270 with a measure of transcript accuracy obtained from prior, e.g., historical, speech recordings.

The measure of transcript accuracy obtained from prior speech recordings can be based on speech recordings of the same person 210 to assess a change in unclear pronunciation of the person 210 and/or to track on or more of a level of language development of the person 210. Unclear pronunciation relates to word-finding difficulties, other types of language attrition, or incoherent speech. Tracking changes in pronunciation of a person 210 over time is a key use case for digital biomarkers.

The method 100 optionally further comprises the step of assessing, based on the change in unclear pronunciation, a change in a cognitive status of the person 210 and/or to track on or more of a cognitive or behavior disorder of the person 210 and autism spectrum disorder, ASD.

The measure of transcript accuracy obtained from prior speech recordings can be prior speech recordings from one or more persons different from the person 210, where the one or more persons different from the person 210 preferably belong to age-, gender-, and education-matched healthy controls, i.e. a control group of people with the same gender and same or similar age and education etc. In this example, the comparison enables determining unclear pronunciation as a pronunciation impairment of the person 210 and/or to track one or more of a level of language development or pronunciation accuracy of the person 210 and a motor or behavioral impairment affecting pronunciation of the person 210. In the use case of comparing with prior speech recordings from other persons, the focus is on pronunciation compared to the general population, e.g., for diagnosis ("pronunciation impairment detected") or therapy ("pronunciation accuracy is back to normal"). In those use cases, the comparison would be made to scores obtained from age-, gender- and education-matched healthy controls, not to prior recordings of the same person.

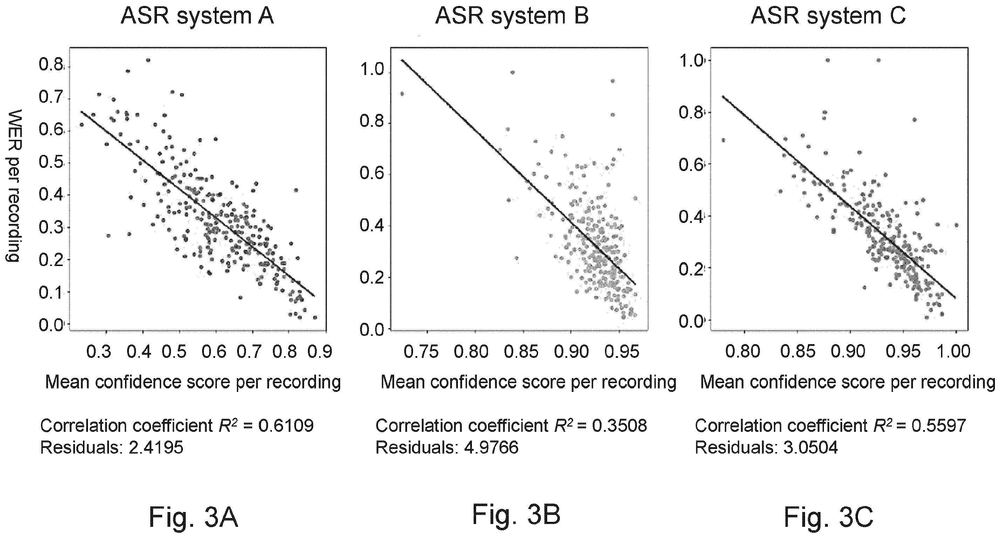

Figs. 3A to 3C illustrate plots of statistical data showing WER per speech recording vs. a conventional mean confidence score per recording created by three different ASR services. The plots in Figs. 3A to 3C will be used to demonstrate shortcomings of conventional WER estimation based on ASR confidence scores.

Commercial ASR services produce confidence scores for each word in a transcript. Figs. 3A, 3B, and 3C show experimental data for three different ASR services respectively and demonstrate that the mean confidence scores are not good predictors of errors, specifically the WER, in automatically generated transcripts, when the transcript is compared to a human transcription. In each Figure, a point cloud is shown, wherein each dot of the point cloud represents a transcription experiment. For each experiment, the y-coordinate shows the true WER of the transcript as created by the ASR service to which the Figure corresponds. WER values are non-negative numbers, wherein lower values represent a higher quality transcription. The x-coordinate represents the mean confidence score across all words of a transcription in a recording. Mean confidence scores range between 0.0 and 1.0, wherein higher values respect higher confidence, suggesting a lower WER.

The plots in Figs. 3A, 3B, and 3C show the following: First, the correlation is not particularly high, indicating *R²* correlation coefficients between 0.3508 and 0.6109. Second, the mean confidence scores greatly vary across different ASR services. In summary, these experimental results show that the mean confidence score is not a reliable estimation of the true WER rate.

Figs. 4A to 4C illustrate plots of statistical data showing WER per recording vs. a difference measure between two automatically generated transcripts, namely a first transcript 250 of an original speech recording 220 and a second transcript 260 of a perturbed speech recording 240, according to the present disclosure, using three different ASR services. The transcript mismatch ratio 270 determined in step 150 provides an estimation of a WER of the transcript as shown with reference to Fig. 4 and discussed in the following. The plots in Figs. 4A to 4C will be used to demonstrate the advantages of WER estimation according to technologies of the present disclosure.

Figs. 4A, 4B, and 4C show experimental data obtained from using ASR system A, B, and C respectively. Each dot in the graphs represents one experiment, which means that each dot corresponds to a speech recording for which a reference manual transcription is available and the true WER when transcribed using ASR system A, B, or C respectively is known. The WER may take values greater than or equal to 0. The lower the WER, the more accurate the transcription. The true WER of each speech recording is projected on the y-axis. The x-axis shows the difference measure between a first transcription obtained through the respective ASR on the original speech recording and the second transcript on the corresponding perturbed speech recording using the same respective ASR. The difference measure is obtained using the techniques described hereinabove. The lower the difference measure, the fewer differences exist between the first original, and the second, perturbed, transcripts. The difference measure may take values greater than or equal to 0. The perturbation and the original speech recording were combined with a SNR of 20. The perturbation was based on a multi-talker babble speech.

The point clouds in each of the graphs corresponding to the respective ASR service are quite linear, which means that there is a relatively strong correlation between the true WER and the difference measure. Figs 4A, 4B, and 4C indicate *R²* correlation coefficients between 0.6227 and 0.7390 for different ASR services. In other words, the difference measure, also referred to as transcript mismatch ratio or ASR reliability measure, is an accurate estimator for the WER. Furthermore, the difference measure is a reliable predictor of ASR accuracy, i.e., the difference measure is mostly independent of the specific ASR technology used. In other words, the ASR reliability measure, expresses how well the given speech recording may be handled by multiple different ASR technologies.

Since the difference measure has a strong correlation with the WER, and since it is not tailored to the particular ASR technology that is used, the measure is reliable indicator of the presence of unclear pronunciation in an original speech recording. In addition, for a particular speech recording, the difference measure can be determined according to the methods presented herein even when the real WER for the original recording on the ASR that is used is not known.

The technology described herein, which includes adding a perturbation, such as speech-like noise, is dependent on how the ASR deals with this perturbation. Specifically, the effectiveness of determining an accuracy of automatically transcribed pathological speech according to the technologies presented herein relies on the ASR's difficulty of recognizing speech in noise. In other words, a future imaginary and more advanced ASR system may be able to perfectly separate a speaker's voice from that of the additive noise / perturbation and could render the methods of determining an accuracy of automatically transcribed pathological speech presented herein inoperable.

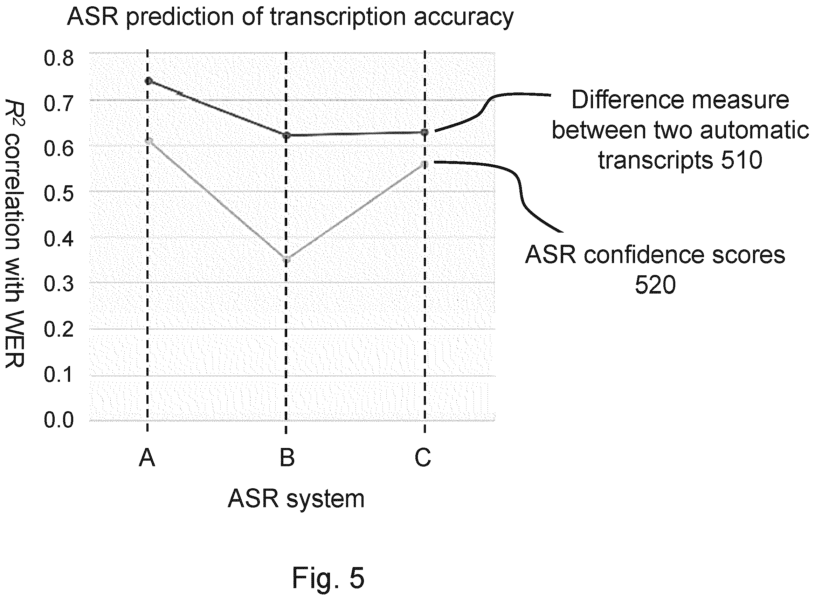

Fig. 5 shows a chart plot comparing the prediction of transcription accuracy based on conventional methods of mean confidence scores with the method according to the present disclosure for three different ASR services. Fig. 5 effectively summarizes the plots shown in Fig. 3 and Fig. 4 with the following result: The difference measure between two automatically generated transcripts, also referred to as transcript mismatch ratio, provided by the technology disclosed herein is a more precise estimator of the true WER compared to the conventional WER estimators based on confidence scores of different ASR systems. This is apparent since curve 510 is above curve 520 in Fig. 5. Furthermore, the estimates according to the disclosed technology are more reliable and not tailored to the underlying ASR implementation. This is apparent, since graph 510 is flatter than graph 520, which shows great variation in the R² correlation across different ASR systems.

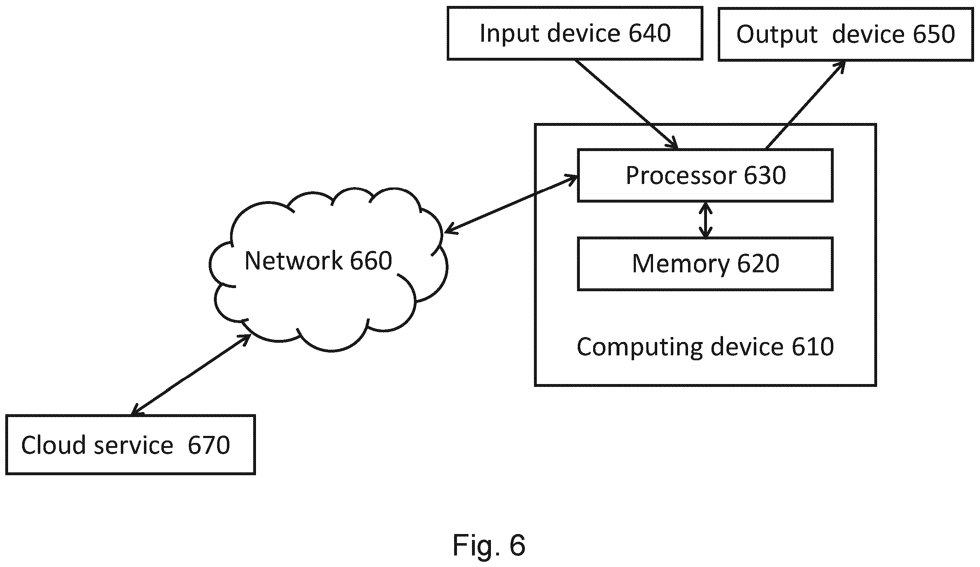

Fig. 6 shows a schematic block diagram of a data processing device 610 adapted to perform methods for determining an accuracy of automatically transcribed pathological speech. The device 610 includes a memory 620 and a processor 630, the processor being in communication with at least on input device 640, e.g., adapted to obtain a speech recording, and an output device 640, for example adapted to display the result of a transcription and. Device 610 can be a general purpose processing device, with memory 620 containing instructions that can be executed on the processor 630 and in communication with the memory 620, in order to perform methods according to the current disclosure. ASR may be performed by the computing device 610 but may also be performed using a cloud service 670 as explained hereinabove, the cloud service 670 being in communication with the computing device 610 through a conventional computer network 660.

Aspects of this disclosure can be implemented in digital circuits, computer-readable storage media, as one or more computer programs, or a combination of one or more of the foregoing that implement, or include instructions that implement the methods described herein. The computer-readable storage media can be non-transitory, e.g., as one or more instructions executable by a cloud computing platform and stored on a tangible storage device.

In this specification the phrase "configured to" is used in different contexts related to computer systems, hardware, or part of a computer program. When a system is said to be configured to perform one or more operations, this means that the system has appropriate software, firmware, and/or hardware installed on the system that, when in operation, causes the system to perform the one or more operations. When some hardware is said to be configured to perform one or more operations, this means that the hardware includes one or more circuits that, when in operation, receive input and generate output according to the input and corresponding to the one or more operations. When a computer program is said to be configured to perform one or more operations, this means that the computer program includes one or more program instructions that, when executed by one or more computers, causes the one or more computers to perform the one or more operations.

Unless otherwise stated, the foregoing alternative examples are not mutually exclusive, but may be implemented in various combinations to achieve unique advantages. In the foregoing description, the provision of the examples described, as well as clauses phrased as "such as," "including" and the like, should not be interpreted as limiting embodiments to the specific examples; rather, the examples are intended to illustrate only one of many possible embodiments.

## Claims

1. A computer-implemented method (100) for determining an accuracy of automatically transcribed pathological speech comprising:
recording (110) speech from a person (210) to obtain an original speech recording (220); said computer-implemented method being **characterised by** further comprising:
combining (120) a perturbation (230) with the original speech recording (220) to obtain a perturbed speech recording (240);
performing (130) automatic speech recognition, ASR, on the original speech recording (220) to obtain a first transcript (250);
performing (140) automatic speech recognition on the perturbed speech recording (240) to obtain a second transcript (260);
comparing (150) the first transcript (250) with the second transcript (260) to quantify a mismatch between the first transcript (250) and the second transcript (260), wherein quantifying a mismatch between the first transcript and the second transcript includes determining a difference as a measure of accuracy (270) of the first transcript (250).

2. The method (100) according to claim 1, wherein the perturbation (230) is one or more of:
additive noise,
multiplicative noise,
reverberation,
decomposing the original speech recording (220) into a parametric representation and re-synthesizing it after varying one or more of the parameters, and
speech-like noise (230) obtained by combining fragments of speech from one or more different voices.

3. The method (100) according to claim 1, wherein a signal-to-noise ratio when combining (120) the perturbation (230) with the original speech recording (220) is larger than 0 and lower than 40 dB.

4. The method (100) according to claim 3, wherein the signal-to-noise ratio is determined by:
performing (130) ASR on each of one or more test speech recordings for which a word error rate is known to obtain one or more original test transcripts;
iteratively performing the steps of
combining (120) the perturbation (230) with each of the test speech recording at a test signal-to-noise ratio to obtain one or more perturbed test speech recordings;
performing (130) ASR on each of the perturbed test speech recordings to obtain one or more perturbed test transcripts,
comparing (150) the original test transcripts (250) with the respective perturbed test transcripts to determine a transcript mismatch measure;
varying the test signal-to-noise ratio based on the transcript mismatch measure;
until a maximum correlation between the transcript mismatch measure and the known word error rate is achieved, wherein the signal-to-noise ratio is determined based on a current test signal-to-noise ratio.

5. The method (100) according to claim 1, where the automatic speech recognition on the original and on the perturbed speech recording is performed using a same ASR technology (280, 290).

6. The method (100) according to claim 1, further comprising the step:
comparing (160) the measure of transcript accuracy (270) with a measure of transcript accuracy obtained from prior speech recordings of the same person (210) to assess a change in unclear pronunciation of the person (210) and/or to track a level of language development of the person (210).

7. The method (100) according to claim 6, further comprising the step:
assessing, based on the change in unclear pronunciation and/or language development, a change in a cognitive status of the person (210) and/or
to track one or more of
a cognitive or behavior disorder of the person (210) and
autism spectrum disorder, ASD.

8. The method (100) according to claim 1, further comprising the step:
comparing (160) the measure of transcript accuracy (270) with a measure of transcript accuracy obtained from speech recordings from one or more persons different from the person (210)
to determine unclear pronunciation as a pronunciation impairment of the person (210) and/or
to track one or more of
a level of language development or pronunciation accuracy of the person (210) and
a motor or behavioral impairment affecting pronunciation of the person (210),
wherein the one or more persons different from person (210) preferably belong to age-, gender-, and education-matched healthy controls.

9. The method (100) according to claim 6, wherein the unclear pronunciation relates to word-finding difficulties, other types of language attrition, or incoherent speech.

10. The method (100) according to claim 1, wherein the speech is pathological due to pronunciation impairments of the person (210).

11. The method (100) according to claim 1, wherein pronunciation impairments relate to individual words and/or comprise one or more of hypoarticulation, hyperarticulation, slurred speech, stutter, and mumble.

12. A data processing device comprising a processor adapted to perform the steps of the method for determining an accuracy of automatically transcribed pathological speech of claim 1.

13. A computer program comprising instructions that, when the program is executed by a computer, cause the computer to carry out the steps of the method for determining an accuracy of automatically transcribed pathological speech of claim 1.

14. A computer-readable medium comprising instructions that, when executed by a computer, cause the computer to carry out the method for determining an accuracy of automatically transcribed pathological speech of claim 1.

## Patentansprüche

1. Computerimplementiertes Verfahren (100) zum Bestimmen einer Genauigkeit von automatisch transkribierter pathologischer Sprache, umfassend:
Aufzeichnen (110) von Sprache von einer Person (210), um eine ursprüngliche Sprachaufzeichnung (220) zu erhalten; wobei das computerimplementierte Verfahren **dadurch gekennzeichnet ist, dass** es ferner Folgendes umfasst:
Kombinieren (120) einer Perturbation (230) mit der ursprünglichen Sprachaufzeichnung (220), um eine perturbierte Sprachaufzeichnung (240) zu erhalten;
Durchführen (130) einer automatischen Spracherkennung, ASR, an der ursprünglichen Sprachaufzeichnung (220), um ein erstes Transkript (250) zu erhalten;
Durchführen (140) einer automatischen Spracherkennung an der perturbierten Sprachaufzeichnung (240), um ein zweites Transkript (260) zu erhalten;
Vergleichen (150) des ersten Transkripts (250) mit dem zweiten Transkript (260), um eine Nichtübereinstimmung zwischen dem ersten Transkript (250) und dem zweiten Transkript (260) zu quantifizieren, wobei das Quantifizieren einer Nichtübereinstimmung zwischen dem ersten Transkript und dem zweiten Transkript das Bestimmen einer Differenz als ein Maß der Genauigkeit (270) des ersten Transkripts (250) einschließt.

2. Verfahren (100) nach Anspruch 1, wobei die Perturbation (230) eine oder mehrere der folgenden ist:
additives Rauschen,
multiplikatives Rauschen,
Nachhall,
Zerlegen der ursprünglichen Sprachaufzeichnung (220) in eine parametrische Darstellung und Neusynthetisieren derselben nach dem Variieren eines oder mehrerer der Parameter, und
sprachähnliches Rauschen (230), das durch Kombinieren von Sprachfragmenten von einer oder mehreren verschiedenen Stimmen erhalten wird.

3. Verfahren (100) nach Anspruch 1, wobei ein Signal-Rausch-Verhältnis beim Kombinieren (120) der Perturbation (230) mit der ursprünglichen Sprachaufzeichnung (220) größer als 0 und kleiner als 40 dB ist.

4. Verfahren (100) nach Anspruch 3, wobei das Signal-Rausch-Verhältnis bestimmt wird durch:
Durchführen (130) einer ASR an jeder der einen oder mehreren Testsprachaufzeichnungen, für die eine Wortfehlerrate bekannt ist, um ein oder mehrere ursprüngliche Testtranskripte zu erhalten;
iteratives Durchführen der folgenden Schritte:
Kombinieren (120) der Perturbation (230) mit jeder der Testsprachaufzeichnungen bei einem Test-Signal-Rausch-Verhältnis, um eine oder mehrere perturbierte Testsprachaufzeichnungen zu erhalten;
Durchführen (130) einer ASR an jeder der perturbierten Testsprachaufzeichnungen, um ein oder mehrere perturbierte Testtranskripte zu erhalten;
Vergleichen (150) der ursprünglichen Testtranskripte (250) mit den jeweiligen perturbierten Testtranskripten, um ein Maß für die Nichtübereinstimmung der Transkripte zu ermitteln;
Variieren des Test-Signal-Rausch-Verhältnisses basierend auf dem Maß für die Nichtübereinstimmung der Transkripte;
bis eine maximale Korrelation zwischen dem Maß für die Nichtübereinstimmung der Transkripte und der bekannten Wortfehlerrate erreicht ist, wobei das Signal-Rausch-Verhältnis basierend auf einem aktuellen Test-Signal-Rausch-Verhältnis bestimmt wird.

5. Verfahren (100) nach Anspruch 1, wobei die automatische Spracherkennung an der ursprünglichen und an der perturbierten Sprachaufzeichnung unter Verwendung derselben ASR-Technologie (280, 290) durchgeführt wird.

6. Verfahren (100) nach Anspruch 1, ferner umfassend den folgenden Schritt:
Vergleichen (160) des Maßes für die Transkriptgenauigkeit (270) mit einem Maß für die Transkriptgenauigkeit, das aus früheren Sprachaufzeichnungen derselben Person (210) erhalten wurde,
um eine Änderung der unklaren Aussprache der Person (210) zu beurteilen und/oder
um einen Grad der Sprachentwicklung der Person (210) zu verfolgen.

7. Verfahren (100) nach Anspruch 6, ferner umfassend den folgenden Schritt:
Beurteilen einer Änderung eines kognitiven Status der Person (210) basierend auf der Änderung der unklaren Aussprache und/oder der Sprachentwicklung und/oder
um eines oder mehreres von
einer kognitiven oder Verhaltensstörung der Person (210) und
Autismus-Spektrum-Störung, ASD, zu verfolgen.

8. Verfahren (100) nach Anspruch 1, ferner umfassend den folgenden Schritt:
Vergleichen (160) des Maßes für die Transkriptgenauigkeit (270) mit einem Maß für die Transkriptgenauigkeit, das aus Sprachaufzeichnungen von einer oder mehreren Personen erhalten wurde, die sich von der Person (210) unterscheiden,
um die unklare Aussprache als eine Ausspracheschwäche der Person (210) zu bestimmen und/oder
um eines oder mehreres von
einem Grad der Sprachentwicklung oder Aussprachegenauigkeit der Person (210) und
einer motorischen oder Verhaltensbeeinträchtigung, die die Aussprache der Person (210) beeinflusst, zu verfolgen,
wobei die eine oder die mehreren Personen, die sich von der Person (210) unterscheiden, vorzugsweise zu gesunden Kontrollen gleichen Alters, Geschlechts und gleicher Bildung gehören.

9. Verfahren (100) nach Anspruch 6, wobei sich die unklare Aussprache auf Schwierigkeiten bei der Wortfindung, andere Arten von Sprachattrition oder inkohärente Sprache bezieht.

10. Verfahren (100) nach Anspruch 1, wobei die Sprache aufgrund von Ausspracheschwächen der Person (210) pathologisch ist.

11. Verfahren (100) nach Anspruch 1, wobei sich Ausspracheschwächen auf einzelne Wörter beziehen und/oder eine oder mehrere von Hypoartikulation, Hyperartikulation, undeutliche Sprache, Stottern und Nuscheln umfassen.

12. Datenverarbeitungsvorrichtung, umfassend einen Prozessor, der dafür ausgelegt ist, die Schritte des Verfahrens zum Bestimmen einer Genauigkeit von automatisch transkribierter pathologischer Sprache nach Anspruch 1 durchzuführen.

13. Computerprogramm, umfassend Anweisungen, die, wenn das Programm von einem Computer ausgeführt wird, den Computer veranlassen, die Schritte des Verfahrens zum Bestimmen einer Genauigkeit von automatisch transkribierter pathologischer Sprache nach Anspruch 1 auszuführen.

14. Computerlesbares Medium, umfassend Anweisungen, die, wenn sie von einem Computer ausgeführt werden, den Computer veranlassen, das Verfahren zum Bestimmen einer Genauigkeit von automatisch transkribierter pathologischer Sprache nach Anspruch 1 auszuführen.

## Revendications

1. Procédé (100) mis en œuvre par ordinateur pour déterminer une précision de la parole pathologique transcrite automatiquement comprenant :
l'enregistrement (110) de la parole d'une personne (210) pour obtenir un enregistrement de parole d'origine (220) ; ledit procédé mis en œuvre par ordinateur étant **caractérisé en ce qu'**il comprend en outre :
la combinaison (120) d'une perturbation (230) avec l'enregistrement de parole d'origine (220) pour obtenir un enregistrement de parole perturbé (240) ;
la réalisation (130) d'une reconnaissance automatique de la parole, ASR, sur l'enregistrement de parole d'origine (220) pour obtenir une première transcription (250) ;
la réalisation (140) d'une reconnaissance automatique de la parole sur l'enregistrement de parole perturbé (240) pour obtenir une seconde transcription (260) ;
la comparaison (150) de la première transcription (250) avec la seconde transcription (260) pour quantifier une disparité entre la première transcription (250) et la seconde transcription (260), dans lequel la quantification d'une disparité entre la première transcription et la seconde transcription comprend la détermination d'une différence en tant que mesure de précision (270) de la première transcription (250).

2. Procédé (100) selon la revendication 1, dans lequel la perturbation (230) est un ou plusieurs parmi :
un bruit additif,
un bruit multiplicatif,
une réverbération,
une décomposition de l'enregistrement de parole d'origine (220) en une représentation paramétrique et une resynthèse de celui-ci après variation d'un ou de plusieurs des paramètres, et
un bruit semblable à la parole (230) obtenu en combinant des fragments de parole d'une ou de plusieurs voix différentes.

3. Procédé (100) selon la revendication 1, dans lequel un rapport signal sur bruit lors de la combinaison (120) de la perturbation (230) avec l'enregistrement de parole d'origine (220) est supérieur à 0 et inférieur à 40 dB.

4. Procédé (100) selon la revendication 3, dans lequel le rapport signal sur bruit est déterminé en :
réalisant (130) une ASR sur chacun des un ou plusieurs enregistrements de parole d'essai pour lesquels un taux d'erreur de mots est connu pour obtenir une ou plusieurs transcriptions d'essai d'origine ;
réalisant de manière itérative les étapes de
combinaison (120) de la perturbation (230) avec chacun des enregistrements de parole d'essai à un rapport signal sur bruit d'essai pour obtenir un ou plusieurs enregistrements de parole d'essai perturbés ;
réalisation (130) d'une ASR sur chacun des enregistrements de parole d'essai perturbés pour obtenir une ou plusieurs transcriptions d'essai perturbées,
comparaison (150) des transcriptions d'essai d'origine (250) avec les transcriptions d'essai perturbées respectives pour déterminer une mesure de disparité de transcription ;
variation du rapport signal sur bruit d'essai en se basant sur la mesure de disparité de transcription ;
jusqu'à ce qu'une corrélation maximale entre la mesure de disparité de transcription et le taux d'erreur de mots connu soit obtenue, dans lequel le rapport signal sur bruit est déterminé en se basant sur un rapport signal sur bruit d'essai actuel.

5. Procédé (100) selon la revendication 1, où la reconnaissance automatique de la parole sur l'enregistrement de parole d'origine et sur l'enregistrement de parole perturbé est réalisée en utilisant une même technologie d'ASR (280, 290).

6. Procédé (100) selon la revendication 1, comprenant en outre l'étape :
comparaison (160) de la mesure de précision de transcription (270) avec une mesure de précision de transcription obtenue à partir d'enregistrements de parole antérieurs de la même personne (210)
pour évaluer un changement de prononciation peu claire de la personne (210) et/ou
pour suivre un niveau de développement du langage de la personne (210).

7. Procédé (100) selon la revendication 6, comprenant en outre l'étape :
évaluation, en se basant sur le changement de prononciation peu claire et/ou de développement du langage, d'un changement d'un état cognitif de la personne (210) et/ou
pour suivre un ou plusieurs parmi
un trouble cognitif ou comportemental de la personne (210) et
un trouble du spectre de l'autisme, TSA.

8. Procédé (100) selon la revendication 1, comprenant en outre l'étape :
comparaison (160) de la mesure de précision de transcription (270) avec une mesure de précision de transcription obtenue à partir d'enregistrements de parole d'une ou de plusieurs personnes différentes de la personne (210)
pour déterminer une prononciation peu claire en tant que trouble de la prononciation de la personne (210) et/ou
pour suivre un ou plusieurs parmi
un niveau de développement du langage ou de précision de prononciation de la personne (210) et
un trouble moteur ou comportemental affectant la prononciation de la personne (210),
dans lequel la ou les personnes différentes de la personne (210) appartiennent de préférence à des témoins sains appariés en âge, sexe et éducation.

9. Procédé (100) selon la revendication 6, dans lequel la prononciation peu claire concerne des difficultés à trouver des mots, d'autres types d'attrition langagière ou une parole incohérente.

10. Procédé (100) selon la revendication 1, dans lequel la parole est pathologique du fait des troubles de la prononciation de la personne (210).

11. Procédé (100) selon la revendication 1, dans lequel les troubles de la prononciation concernent des mots individuels et/ou comprennent un ou plusieurs parmi l'hypoarticulation, l'hyperarticulation, la parole brouillée, le bégaiement et le marmonnement.

12. Dispositif de traitement de données comprenant un processeur adapté pour réaliser les étapes du procédé pour déterminer une précision de la parole pathologique transcrite automatiquement selon la revendication 1.

13. Programme informatique comprenant des instructions qui, lorsque le programme est exécuté par un ordinateur, amènent l'ordinateur à mettre en œuvre les étapes du procédé pour déterminer une précision de la parole pathologique transcrite automatiquement selon la revendication 1.

14. Support lisible par ordinateur comprenant des instructions qui, lorsqu'elles sont exécutées par un ordinateur, amènent l'ordinateur à mettre en œuvre le procédé pour déterminer une précision de la parole pathologique transcrite automatiquement selon la revendication 1.
